(19) Europäisches Patentamt
European Patent Office
Office européen des brevets

(11) **EP 4 702 984 A1**

(12) **EUROPEAN PATENT APPLICATION**

(43) Date of publication:
04.03.2026 Bulletin 2026/10

(21) Application number: 25198615.4

(22) Date of filing: 28.08.2025

(51) International Patent Classification (IPC):
*A61K 36/8988* (2006.01)  *A61K 36/11* (2006.01)
*A61K 36/07* (2006.01)  *A61K 31/714* (2006.01)
*A61K 36/074* (2006.01)  *A23L 33/105* (2016.01)
*A61P 25/00* (2006.01)  *A61P 25/28* (2006.01)
*A61P 29/00* (2006.01)  *A61K 36/45* (2006.01)

(52) Cooperative Patent Classification (CPC):
(C-Sets available)
A61P 25/00; A23L 33/105; A61K 31/7034;
A61K 36/07; A61K 36/071; A61K 36/074;
A61K 36/11; A61K 36/45; A61K 36/8988;
A61P 25/28; A61P 29/00          (Cont.)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 28.08.2024 IT 202400019315

(71) Applicant: Nutra Futura S.r.l.
20155 Milano (IT)

(72) Inventor: UBERTI, Francesca
28838 Stresa (Verbano-Cusio-Ossola) (IT)

(74) Representative: Delbarba, Andrea
Bugnion S.p.A.
Viale Lancetti, 17
20158 Milano (IT)

(54) **A COMPOSITION COMPRISING AN EQUISETUM EXTRACT AND USES THEREOF**

(57) The present invention relates to a composition comprising or, alternatively, consisting of an extract of a plant belonging to the genus *Equisetum* and at least one further ingredient selected in the group comprising or, alternatively, consisting of i) an extract of a plant belonging to the genus *Gastrodia,* ii) at least one extract of a fungus selected from those belonging to a species selected from the group comprising or, alternatively, consisting of *Grifola frondosa, Ganoderma lucidum, Hericium erinaceus, Agaricus blazei Murr* and *Inonotus obliquus* (Chaga), or mixtures thereof between (i) and (ii). Furthermore, the present invention relates to pharmaceutical and/or nutraceutical compositions comprising said composition and the use thereof in therapy, in particular in a pain treatment method.

Fig. 1

Processed by Luminess, 75001 PARIS (FR)

(Cont. next page)

EP 4 702 984 A1

(52) Cooperative Patent Classification (CPC): (Cont.)

C-Sets
**A61K 31/7034, A61K 2300/00;**
**A61K 36/07, A61K 2300/00;**
**A61K 36/071, A61K 2300/00;**
**A61K 36/074, A61K 2300/00;**
**A61K 36/11, A61K 2300/00;**
**A61K 36/45, A61K 2300/00;**
**A61K 36/8988, A61K 2300/00**

**Description**

The present invention relates to a composition comprising or, alternatively, consisting of an extract of a plant belonging to the genus *Equisetum* and the uses thereof in therapy.

**STATE OF THE ART**

In the coming decades, the gradual and progressive aging of the population in the Western world will lead to an increase in the number of elderly people who suffer from dementia associated with painful conditions. In fact, aging represents the largest risk factor for the onset of both cognitive decline (dementia in the most severe cases) and pain, as well as their coexistence.

Cognitive decline and pain are complex phenomena, considering the different subtypes of cognitive decline and the different ways in which pain is expressed (nociceptive, neuropathic or central pain).

Nociceptive pain is the process whereby a harmful stimulus is perceived at a peripheral level by nociceptors (peripheral nerve endings) and transmitted to the central nervous system.

Neuropathic pain is caused by damage to or a dysfunction of the peripheral or central nervous system, rather than by stimulation of pain receptors. Neuropathic pain is distinguished from somatic pain, in which the damage instead affects the peripheral receptor system. Examples of neuropathic pain are phantom limb pain, which may occur after an amputation, pain in the so-called complex regional pain syndrome or reflex sympathetic dystrophy, and pain due to central nervous system injuries. The characteristics of this pain vary from patient to patient, but in general there are sensations of constant burning or electric shocks.

The molecular mechanisms of neuropathic pain are complex and involve changes at the level of the nociceptors and peripheral nerves, at the level of the dorsal root ganglion and in the nociceptive pathways of the central nervous system and in terminal structures.

At the level of the peripheral nerves and nociceptors, the injury causes inflammation and activation of the cation channels, in particular the sodium channels. These alterations reduce the activation threshold and increase the response to noxious stimuli. In chronic states, the peripheral nerve continuously triggers ectopic nociceptive signals to the central nervous system. Neuropathic pain is often difficult to treat: analgesics, including even morphine, are often ineffective. Recourse is often had to palliative therapies with antidepressants, or more invasive techniques such as anaesthetic blocks, neurostimulations or surgical interventions, but specific treatments do not yet exist, and this is one of the most frustrating problems of pain therapy.

Furthermore, neuropathic pain is often associated with aging, a decline in cognitive functions and, in the most severe cases, dementia.

Cognitive and mental decline mean a deterioration of cognitive functions (mental activities, ability to reason, memory), as well as behavioural and emotional ones, such as to interfere with everyday activities.

Cognitive decline can vary from a mild to moderate or severe form based on the degree of alteration of cognitive functions. Severe cognitive decline, which is often associated with aging and/or the presence of diseases (such as neurodegenerative diseases, developmental disabilities, brain tumours, strokes or traumatic brain injuries) may lead to a loss of the ability to understand, speak or write, with a consequent inability to live independently.

There is thus a high demand on the part of society for suitable treatments for conditions associated with brain aging which are simultaneously also capable of acting on neuropathic pain, in order to treat, with a single therapeutic product, individuals having neuropathic pain with at least an initial decline in cognitive functions.

In particular, there is a felt need to provide a product to be used in a multimodal therapy, together with other drugs or methods conventionally used in the treatment of neuropathic pain, one which has no side effects and does not interfere with the action of conventionally used drugs. Therefore, it is necessary that this product be composed of natural ingredients and that, advantageously, it can be administered to a broad category of individuals, including immunocompromised individuals.

The technical problem that the present invention addresses is to provide a product for use in the treatment of cognitive decline which is simultaneously useful for treating neuropathic pain. These aims and still others, which will become clear from the detailed description that follows, are achieved by a composition comprising the technical features claimed in the appended claims.

**SUMMARY OF THE INVENTION**

A first aspect of the present invention relates to a composition comprising an extract of a plant belonging to the genus *Equisetum* and at least one further ingredient selected from the group consisting of:

i) an extract of a plant belonging to the genus *Gastrodia,* preferably to the species G. *elata,*

ii) an extract of a fungus belonging to the species *Agaricus blazei Murrill* or *Inonotus obliquus* (Chaga), and mixtures thereof.

In one embodiment, the composition comprises or consists of an extract of the plant belonging to the genus *Equisetum* and an extract of the fungus belonging to the species *Inonotus obliquus* (Chaga).

In one embodiment, the composition comprises or consists of an extract of the plant belonging to the genus *Equisetum* and an extract of the fungus belonging to the species *Agaricus blazei Murrill*.

In one embodiment, the composition comprises or consists of an extract of the plant belonging to the genus *Equisetum* and an extract of a plant belonging to the genus *Gastrodia*. Preferably, the extract of the plant belonging to the genus *Equisetum* belongs to the species *Equisetum L. arvense*.

Preferably, the composition comprises the extract of a plant belonging to the genus *Equisetum* and the extract of the fungus belonging to the species *Inonotus obliquus* (Chaga) in a weight ratio by weight ranging between 1:10 and 1:1, preferably between 1:8 and 1:3.

Preferably, the composition comprises the extract of a plant belonging to the genus *Equisetum* and the extract of the fungus belonging to the species *Agaricus blazei Murrill* in a weight ratio by weight ranging between 1:10 and 1:1, preferably between 1:8 and 1:3.

Preferably, the composition comprises the *Equisetum* extract and the *Gastrodia* extract in a weight ratio by weight ranging between 1:10 and 2:1, preferably between 1:5 and 1:1.

In one embodiment, the composition is formulated for oral administration, preferably as granules, a tablet, capsule, sachet, stick, orodispersible stick, gel, pill, oral ampoule, soluble powder, or syrup.

A second aspect of the present invention relates to the above-described composition for use as a medicament.

A third aspect of the present invention relates to the above-described composition for use in the treatment or prevention of pain.

In one embodiment, the pain is a chronic pain and/or an acute pain.

Preferably, the pain is a nociceptive pain, a neuropathic pain and/or a dysfunctional pain, wherein the neuropathic pain is a central or peripheral neuropathic pain.

In one embodiment, the composition, for the above-described medical uses, is administered in association or in combination with at least one active ingredient or composition for treating pain and/or inflammation in an individual.

A fourth aspect of the present invention relates to a dietary supplement which comprises or consists of the above-described composition.

## DESCRIPTION OF THE FIGURES

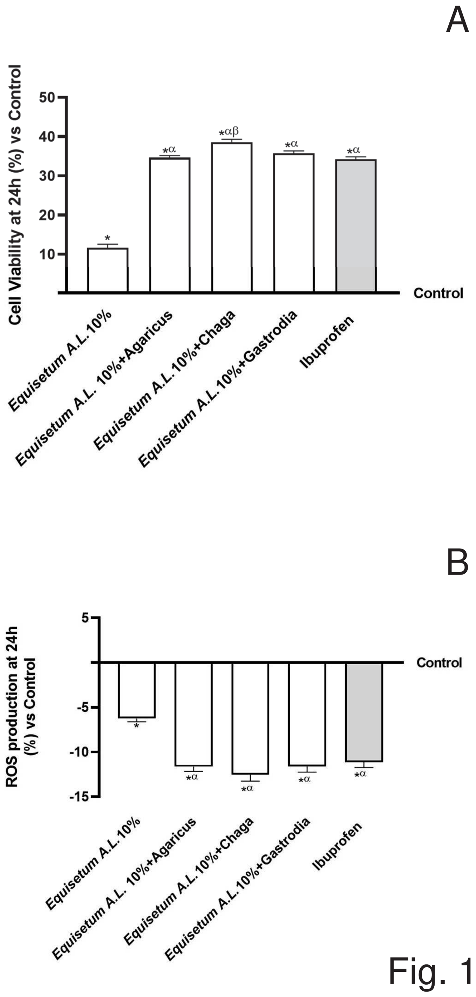

Figure 1 shows the cell viability (A) and the ROS production rate (B) obtained after 24 hours of treatment of the in vitro 3D blood-brain barrier model prepared with CCF-STTG1 astrocyte cells, pericytes and human umbilical vein endothelial cells (HUVECs) with the samples tested in example 1 and indicated in the figure. The data are expressed as the mean $\pm$ SD (%) of 5 independent experiments carried out in triplicate, normalised to the control. * $p < 0.05$ vs control; $\alpha$ $p < 0.05$ vs *Equisetum A.L.* 10%; $\beta$ $p < 0.05$ vs Ibuprofen.

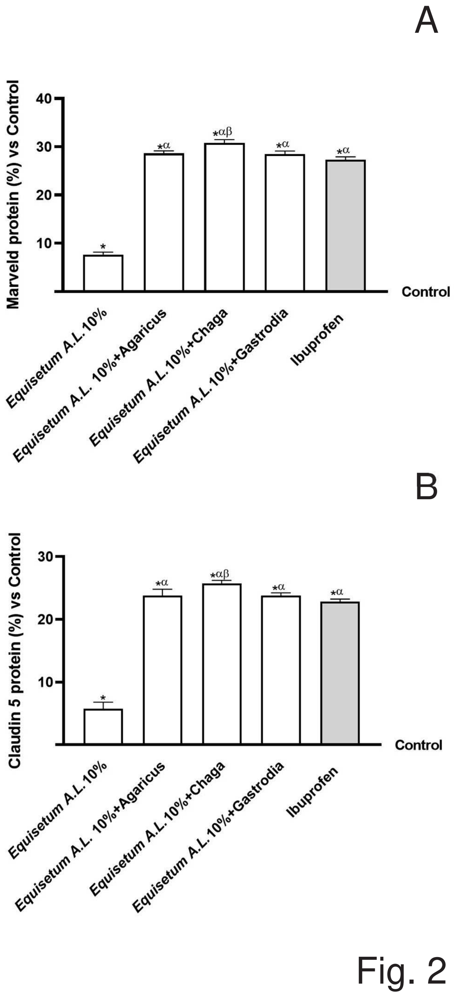

Figure 2 shows the results relating to an analysis of the state of integrity of the blood-brain barrier, in particular by evaluating the activity of the tight junction proteins marveld (A) and claudin-5 (B); evaluation after 24 hours of treatment with the samples tested in example 1 and indicated in the figure. The data are expressed as the mean $\pm$ SD (%) of 5 independent experiments carried out in triplicate, normalised to the control. * $p < 0.05$ vs control; $\alpha$ $p < 0.05$ vs *Equisetum A.L.* 10%; $\beta$ $p < 0.05$ vs Ibuprofen.

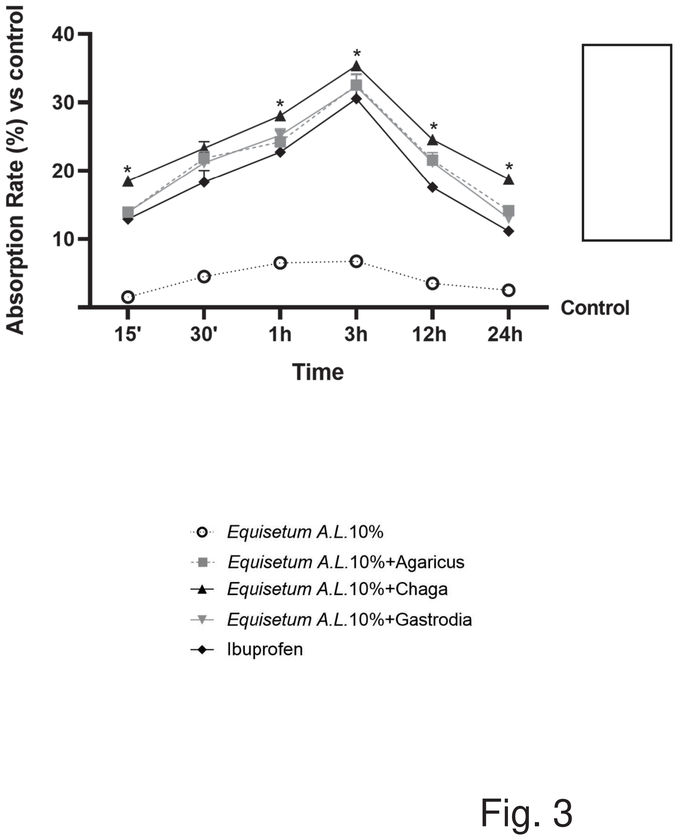

Figure 3 shows the rate of absorption, through the blood-brain barrier, of the samples indicated in the figure. The data are expressed as the mean $\pm$ SD (%) of 5 independent experiments carried out in triplicate, normalised to the control. All the combinations and Ibuprofen $p < 0.05$ vs control; * $p < 0.05$ vs all the other agents.

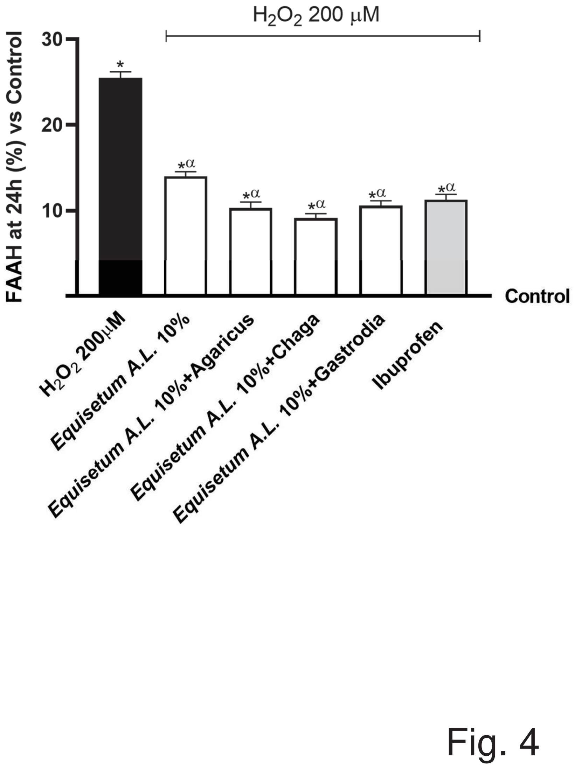

Figure 4 shows the FAAH levels detected after treatment of an organoid previously treated with $200 \mu M$ of $H_2O_2$ for 30 min with the samples indicated in the figure. The data are expressed as the mean $\pm$ SD (%) of 5 independent experiments carried out in triplicate, normalised to the control. * $p < 0.05$ vs control; $\alpha$ $p < 0.05$ vs $H_2O_2$ 200 $\mu M$.

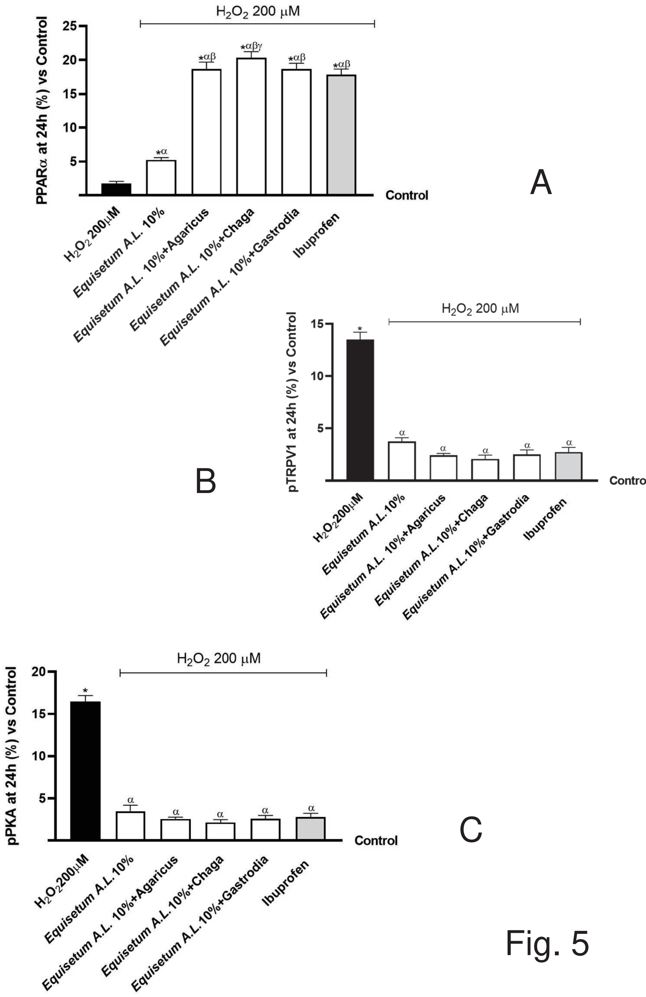

Figure 5 shows the PPAR$\alpha$ (A), pTRPV1 (B) and pPKA (C) levels detected after treatment of an organoid previously treated with $200 \mu M$ of $H_2O_2$ for 30 min with the samples indicated in the figure. The data are expressed as the mean $\pm$ SD (%) of 5 independent experiments carried out in triplicate, normalised to the control. * $p < 0.05$ vs control; $\alpha$ $p < 0.05$ vs $H_2O_2$ 200 $\mu M$; $\beta$ $p < 0.05$ vs *Equisetum A.L.* 10%; $\gamma$ $p < 0.05$ vs Ibuprofen.

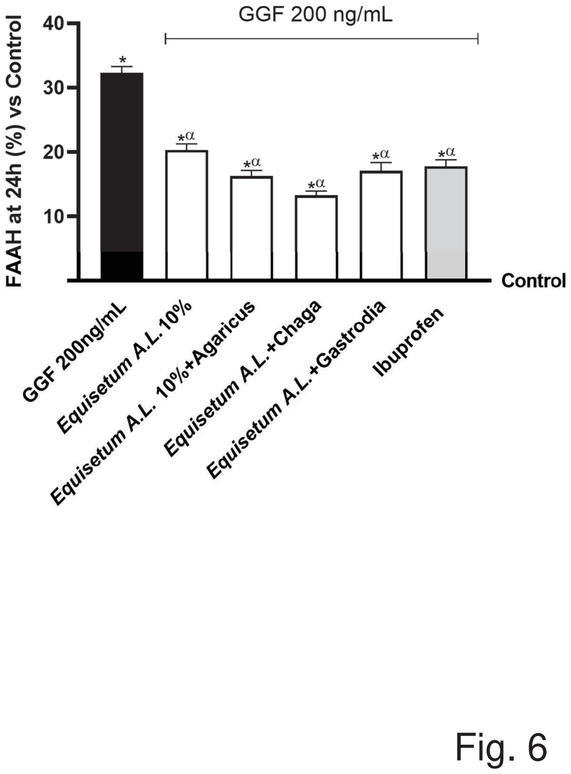

Figure 6 shows the FAAH levels detected after treatment of a 3D peripheral nerve model - previously treated with GGF 200 ng/mL for 14 days - with the samples indicated in the figure. The data are expressed as the mean $\pm$ SD (%) of 5 independent experiments carried out in triplicate, normalised to the control. * $p < 0.05$ vs control; $\alpha$ $p < 0.05$ vs GGF 200 ng/mL.

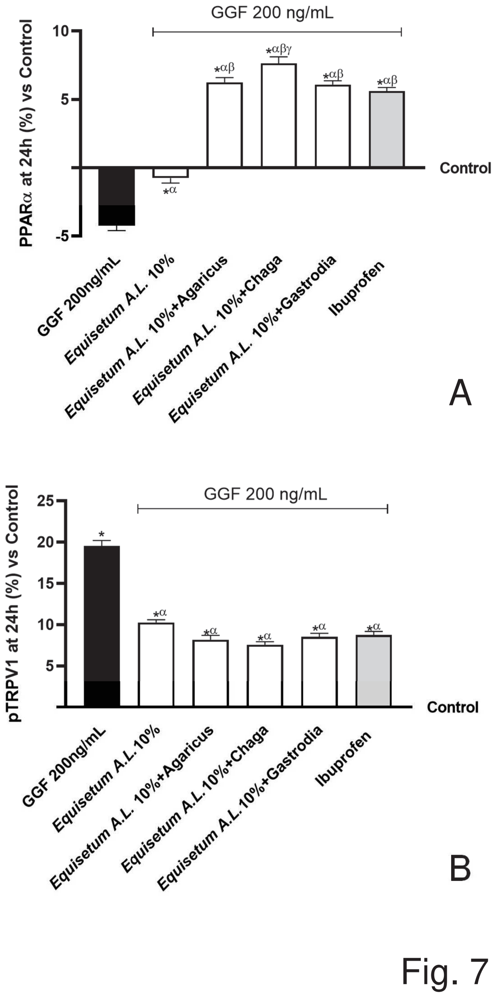

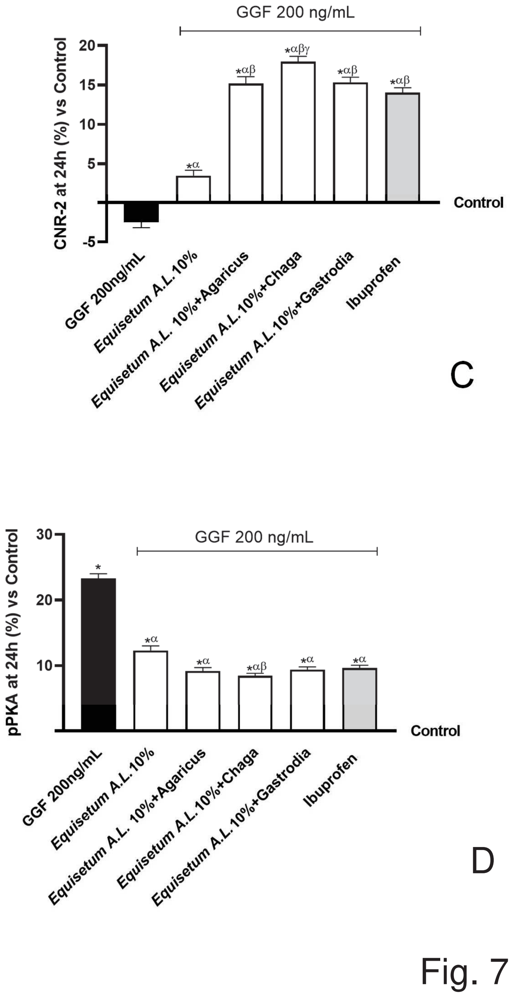

Figure 7 shows the PPAR$\alpha$ (A), pTRPV1 (B), CNR2 (C) and pPKA (D) levels detected after treatment of a 3D peripheral nerve model - previously treated with GGF 200 ng/mL for 14 days - with the samples indicated in the figure. The data are expressed as the mean $\pm$ SD (%) of 5 independent experiments carried out in triplicate, normalised to the control. * p<0.05 vs control; $\alpha$ p<0.05 vs H$_2$O$_2$ 200 $\mu$M; $\beta$ p<0.05 vs *Equisetum A.L.* 10%; $\gamma$ p<0.05 vs Ibuprofen.

## DETAILED DESCRIPTION OF THE INVENTION

A first aspect of the present invention regards a composition comprising an extract of a plant belonging to the genus *Equisetum* and at least one further ingredient selected from the group consisting of:

i) an extract of a plant belonging to the genus *Gastrodia,* preferably to the species G. *elata,*
ii) an extract of a fungus belonging to the species *Agaricus blazei Murrill* or *Inonotus obliquus* (Chaga) and mixtures thereof.

*Equisetum* is a plant belonging to the family *Equisetaceae* and is commonly known by the name of "horsetail".
Fifteen species belong to the genus *Equisetum,* a little less than ten of which are present among Italian flora.
In the context of the present invention, the term *"Equisetum"* includes extracts, preferably aqueous, alcoholic or hydroalcoholic extracts, for example extracts of *Equisetum Arvense L.* In the context of the present invention, in the experimental part that follows and in the appended figures, *Equisetum Arvense L.* can be indicated as *Equisetum A.L.*
The most widely recognised phytochemical constituents of *Equisetum A.L* are flavonoids, phenolic acids, alkaloids, phytosterols, tannins and triterpenoids.
In the context of the present invention, "an *Equisetum* extract" is meant to refer to, and include, any extract (and/or mixtures of extracts) of *Equisetum* obtained from any source with the methods and equipment well known to the person skilled in the art.
Preferably, said *Equisetum* extract is an *Equisetum Arvense L* extract.
For example, the *Equisetum* can be used in the form of an extract, for example, an extract containing from 1% to 40% silica, preferably containing from 5% to 30%, more preferably containing from 7.5% to 20%, even more preferably containing from 10% to 15% silica.
Preferably, the *Equisetum* can be used in any form thereof, for example in powder form, as a fluid extract, mother tincture or macerated.
Preferably the *Equisetum* used in the composition of the invention has a silica content greater than 1%, more preferably greater than 7%, for example a silica content of around 10%, and a content of less than 20%, preferably a content less than 15%.
An example of an *Equisetum Arvense L* dry extract may be represented by a 10% silica dry extract, with a particle size of 95% passing through an 80 mesh sieve and a minimum silica content of 10% as measured by UV.
As shown in the experimental section that follows, *Equisetum Arvense L.* (*Equisetum A.L.*) has revealed to be capable of amplifying the analgesic effect of substances in combination, such as Gastrodia, Agaricus and Chaga extracts, by acting on the mechanisms involved in triggering pain transmission at the level of the central nervous system (CNS) and peripheral nervous system (PNS).
Preferably, the composition according to the invention comprises or consists of an extract of a plant belonging to the genus *Equisetum* and at least one further ingredient selected from the group comprising or, alternatively, consisting of i) an extract of a plant belonging to the genus *Gastrodia,* preferably to the species G. *elata* and ii) at least one extract of a fungus, wherein said fungus is preferably selected from those belonging to the species selected from the group comprising or, alternatively, consisting of *Grifola frondosa, Ganoderma lucidum, Hericium erinaceus, Agaricus blazei Murrill* and *Inonotus obliquus,* or iii) mixtures thereof between (i) and (ii).
*Gastrodia elata* belongs to the family *Orchidaceae,* and is a plant traditionally used in Eastern countries to treat various conditions such as headache, dizziness, spasms, epilepsy, stroke, amnesia and other disorders.
The part of the plant that shows an activity is its rhizome (*Gastrodiae Rhizoma*), from which phenols, polysaccharides, organic acids and sterols, for example, can be isolated. Each of these categories of compounds can contain within them a number of specific compounds or specific chemical entities.
In the context of the present invention "a *Gastrodia elata* extract" is meant to refer to, and include, any extract (and/or mixtures of extracts) of *Gastrodia elata* obtained from any source with the methods and equipment well known to the person skilled in the art.
Gastrodia extract, preferably *Gastrodia elata* extract, is thus meant to include an extract comprising or consisting of one or more of said categories of isolated compounds, each of which may be present in said extract in an amount ranging from zero to 100% by weight, relative to the weight of the extract. For example, said extract can comprise or consist of a composition of said specific compounds or specific chemical entities belonging to one or more of said categories of compounds.

Preferably, the Gastrodia extract can be, for example, a 10:1 *Gastrodia elata* extract, i.e. standardised so as to contain, for example, a concentration 10 times greater than the concentration of active substances present in the tuber of *Gastrodia elata.*

For example, a 10:1 extract means the [drug:extract] ratio (the active ingredients contained in 10 grams of plant correspond to 1 gram of extract).

The *Gastrodia elata* extract is preferably a *Gastrodia elata* dry extract.

Preferably, said extract may be obtained by extraction with an extractive solvent, for example water or a water/alcohol (for example ethyl alcohol or ethanol) solution at a temperature, for example, ranging from 20°C to 70°C. Subsequently, one preferably obtains a powder by drying or by nebulisation with a spray-drying system to yield a nebulised dry extract, wherein, for example, the extract is nebulised in a high-temperature current of steam. The short time of contact between the extract and the steam brings about instant drying without damaging the active ingredients. Highly concentrated extracts are obtained.

Preferably, the *Gastrodia elata* extract, for example, a *Gastrodia elata* dry extract, with a drug:extract ratio of 10:1, can have a phytochemical profile of the following type:

- a total polysaccharide content ranging from 40% to 90%, preferably from 50% to 80%, more preferably from 60% to 70%; for example, alpha-glucans and/or maltodextrins can be present in an amount ranging from greater than zero to less than 10%, preferably from greater than 0.1% to less than 7.5%, more preferably from greater than 2% to less than 5%;
- a triterpene content ranging from 0.5% to 5%, preferably from 1% to 4%, more preferably from 1.5% to 3%;
- a total polyphenol content ranging from 0.05% to 3%, preferably from 0.1% to 2%, more preferably from 0.2% to 1%.

Furthermore, also present in said extract there can be, for example, lignans in an amount ranging from greater than zero to less than 0.01%, for example, less than 0.05%, as well as triterpenes in an amount ranging from greater than zero to less than 0.01%, for example, less than 0.05%. Preferably, said triterpenes and polyphenols can have a low molecular weight and belong to the class of phenolic glycosides.

As a further example, the *Gastrodia elata* extract can be a *Gastrodia elata* rhizome or root extract prepared, for example, by extraction with water and ethanol and having a polysaccharide content (measured with the UV phenol-sulphuric acid method, 490 nm) of from 50% to 90%, preferably from 60% to 80%; a particle size of 80 mesh (min. 95% passing through an 80 mesh sieve) and a bulk density CP <2020> ranging from 0.3 g/ml to 0.6 g/ml.

As a further example, the *Gastrodia elata* extract can be a 10:1 P.E. *Gastrodia elata* rhizome extract (dry extract in the presence of dextrin as an excipient) having a polysaccharide content (measured with the UV phenol-sulphuric acid method, 490 nm) of from 10% to 40%, preferably from 15% to 20%; a particle size of 100 mesh (100% passing through a 100 mesh sieve), a water solubility of 100% at a temperature of 25°C and a tapped density ranging from 0.35 to 0.55 g/cm3.

Preferably, the composition comprises the *Equisetum* extract and the *Gastrodia* extract in a weight ratio by weight ranging between 1:10 and 2:1, preferably between 1:5 and 1:1.

Preferably, the fungi present in the composition are in the form, for example, of extracts. In particular, said extracts can be obtained from any source with the methods and equipment well known to the person skilled in the art.

Preferably, *Grifola frondosa* is a saprophytic-parasitic fungus that grows under chestnut trees and belongs to the family *Meripilaceae.*

For example, a *Grifola frondosa* dry extract is an extract consisting of a powder with a particle size of 95% passing through a 100 mesh sieve and which has a polysaccharide content (active compounds) > 3% as measured by UV.

Preferably, *Ganoderma lucidum* is a parasitic or saprophytic fungus belonging to the family *Ganodermataceae.*

For example, a *Ganoderma lucidum* dry extract consists of a powder with a particle size of 95% passing through an 80 mesh sieve and with a polysaccharide content (active compounds) > 5% as measured by UV.

Preferably, *Hericium erinaceus* is an edible fungus used for medicinal purposes and belonging to the genus *Hericium. Hericium* contains polysaccharides such as, for example, β-glucan, heteroglycans, heteroxylans, and triterpenes such as, for example, hericenone and erinacine, which are present, respectively, in the fruit and in the mycelium.

Preferably, a *Hericium erinaceus* dry extract consists of a powder with a particle size of 95% passing through a 100 mesh sieve and which has a polysaccharide content (active compounds) > 5% as measured by UV.

Preferably, the composition according to the invention comprises at least one extract of a fungus selected from those belonging to a species selected from the group comprising or, alternatively, consisting of *Agaricus blazei Murrill* and *Inonotus obliquus* (*Chaga*)*,* or mixtures thereof.

*Agaricus L.,* 1753 is a genus of basidiomycetous fungi belonging to the family *Agaricaceae* and comprising species of various sizes, such as the famous "champignon", a cultivated mushroom (*Agaricus bisporus*).

For example, an *Agaricus blazei Murrill* dry extract consists of a powder with a particle size of 95% passing through a 100 mesh sieve and which has a polysaccharide content (active compounds) > 3% as measured by UV.

Preferably, the composition according to the invention comprises an *Equisetum* extract and fungi belonging to the species *Agaricus blazei Murrill* in the form of an extract, in a weight ratio by weight ranging from 1:10 to 1:1, preferably from 1:8 to 1:3.

More preferably, the fungus present in the composition according to the invention belongs to the species *Inonotus obliquus* (Chaga).

Preferably, the composition comprises at least one Chaga extract, obtained with methods and equipment known in the industry.

For example, said extract appears as a slightly bitter, brown to light brown powder with a particle size of 95% passing through an 80 mesh sieve. For example, the Chaga dry extract can have a polysaccharide content (active compounds) > 3% as measured by UV.

More preferably, the composition consists of an *Equisetum* extract, in particular an *Equisetum L. arvense* extract, and *Inonotus obliquus* (Chaga).

The composition consisting of an *Equisetum* extract, in particular an *Equisetum L. arvense* extract, and Chaga:

- demonstrated a better potential for maintaining and increasing cell viability and a better antioxidant potential compared to the other combinations and ibuprofen alone;
- demonstrated a better potential for maintaining and enhancing blood-brain barrier function and integrity, as reproduced *in vitro,* compared to the other combinations;
- has the best absorption kinetics;
- gave the best results as regards the activity of the molecular markers correlated to the triggering of pain transmission at the level of the CNS (see example 1) and PNS (see example 2).

Preferably, the composition comprises an *Equisetum* extract and an extract of the fungus belonging to the species *Inonotus obliquus* (Chaga) in a weight ratio by weight ranging from 1:10 to 1:1, preferably from 1:8 to 1:2.

The composition described thus far is prepared by means of the methods and equipment known to the person skilled in the art in the nutraceutical or pharmaceutical field.

A **second** aspect of the present invention relates to a pharmaceutical and/or nutraceutical composition that comprises the composition as described above, together with one or more food or pharmaceutical grade excipients and/or vehicles.

In the present invention, the term "composition" includes pharmaceutical, nutraceutical or food compositions or compositions for medical devices as per Regulation (EU) 745/2017, a dietary supplement and/or a food for special medical purposes (FSMP).

Preferably, the composition of the present invention is present in the pharmaceutical or nutraceutical composition in an amount by weight ranging from 10% to 90%, preferably from 25% to 75%, more preferably from 40% to 60%, whereas said food or pharmaceutical grade excipient and/or vehicle are present in an amount by weight ranging from 90% to 10%, preferably from 75% to 25%, more preferably ranging from 60% to 40% by weight, relative to the total weight of the composition.

Preferably, the composition of the invention can also contain further components, preferably selected from those of natural origin which are useful, for example, for reinforcing cognitive functions. By way of example, the composition can further comprise vitamins, in particular group B vitamins.

The composition may optionally comprise further components such as a bilberry extract and/or a citicoline, preferably in the presence or absence of at least one group B vitamin.

Preferably, the composition may optionally comprise *Equisetum L. arvense,* Chaga and a bilberry extract.

The person skilled in the art is perfectly capable of selecting the most suitable vehicles and excipients for preparing the composition according to the invention.

Examples of excipients used to prepare solid food, nutraceutical and/or pharmaceutical compositions include, for example, lactose, sucrose, starch, talc, cellulose, dextrins, kaolin, calcium carbonate, stearic acid or magnesium stearate, lactose, polyethylene glycol, mannitol, sorbitol, chelating agents, anti-caking agents, sweetening agents, preservative agents and flavouring agents.

The composition of the invention can be prepared by mixing the individual components, and any conventional excipients and/or vehicles. In particular, said composition is prepared by means of methods and equipment known to the person skilled in the art in the nutraceutical or pharmaceutical field.

The individual to be treated with the composition of the invention is preferably a mammal, more preferably a human being.

The composition of the invention is a composition for oral use, preferably a composition in a solid state, preferably formulated in dosage units.

"Solid state" means that the composition can exist in the form of granules or powders. The granular or powder compositions are mixed with pharmacologically acceptable additives and excipients in order to provide a final product, such as, for example, a supplement product, a medical device or a pharmaceutical composition. The final product can be in pharmaceutical dosage units, such as, for example, granules in a sachet, tablets, or capsules.

The tablets can be coated or film-coated with one or more layers of a coating or film capable of passing through the gastric barrier, according to the known methods.

The gel capsules can consist of hard gelatine or soft gelatine or soft gel.

The solid compositions of the invention can contain, as mentioned, conventional physiologically acceptable excipients and vehicles such as diluents, bulking agents, binders, disaggregating agents, flow aids, lubricants, etc. Non-limiting examples of suitable vehicles and excipients are described in Remington: The Science and Practice of Pharmacy, 21st Ed., Lippincott, Williams & Wilkins.

The composition is well tolerated and may thus be used in therapy.

In one embodiment, the composition is formulated as granules, a tablet, capsule, sachet, stick, orodispersible stick, gel, pill, oral ampoule, soluble powder or syrup.

A **third** aspect of the present invention relates to the composition described above in detail for use as a medicament.

A **fourth** aspect of the present invention relates to the composition described above in detail for use in the treatment or prevention of pain.

Preferably, the composition is used to treat or prevent chronic pain and/or acute pain. Preferably, the pain is a nociceptive pain, a neuropathic pain and/or a dysfunctional pain, wherein the neuropathic pain is a central or peripheral neuropathic pain.

In one embodiment, the composition is administered to an individual in need thereof in association or in combination with at least one active ingredient or a composition for treating pain and/or inflammation in an individual.

Preferably, the composition is used in the treatment of cognitive decline and/or to slow brain aging.

Preferably, said composition is advantageously administered to an individual suffering from a decline of cognitive functions.

Furthermore, the composition can be administered to an individual who shows the first signs of aging in order to slow brain aging and prevent cognitive decline.

Advantageously, the composition is used individually or in combination with at least one drug or supplement conventionally used in the treatment of cognitive decline.

The present invention also relates to the use of an *Equisetum* extract, preferably of the composition as described above in detail, as a system for delivering biological molecules and/or drugs, preferably as a system for delivering biological molecules and/or drugs through the blood-brain barrier. Preferably, said *Equisetum* extract for use as a system for delivering biological molecules and/or drugs is an *Equisetum L. arvense* extract.

In fact, as shown in the examples that follow, *Equisetum* and the composition as described above in detail is capable of passing through the blood-brain barrier and of amplifying the analgesic effect of substances in combination, by acting on mechanisms involved in triggering pain transmission at the level of the central nervous system (CNS) and peripheral nervous system (PNS).

A further aspect of the present invention relates to a method for treating or preventing pain in an individual. Said method comprises at least a step of administering the composition as described above in detail.

Preferably, the composition is used to treat or prevent chronic pain and/or acute pain. Preferably, the pain is a nociceptive pain, a neuropathic pain and/or a dysfunctional pain, wherein the neuropathic pain is a central or peripheral neuropathic pain.

In one embodiment, the composition is administered to an individual in need thereof in association or in combination with at least one active ingredient or a composition for treating pain and/or inflammation in an individual.

**EXAMPLES**

**Experimental protocol**

With the aim of analysing the effect of an *Equisetum L. Arvense* extract in amplifying the analgesic effect of preferred substances, such as the active compounds contained in Gastrodia, Agaricus and Chaga, the samples indicated in Table 1 below were tested and their effectiveness was tested against ibuprofen (25 $\mu$g/mL).

**Table 1: samples analysed**

| | |
|---|---|
| *Equisetum A.L.* | 10% (50$\mu$g/mL) |
| *Equisetum A.L.* | 10% + Gastrodia (50$\mu$g/mL) |
| *Equisetum A.L.* | 10% + Agaricus (*) (240$\mu$g/mL) |

(continued)

| Equisetum A.L. | 10% + Chaga (250 $\mu$g/mL) |
|---|---|
| (*) e.g. Agaricus blazei Murrill. | |

For the purpose of testing the samples indicated in table 1, an *in vitro* study divided into two phases was carried out.

In the first phase of the experiment, the samples were examined to assess the effect after passage through an in vitro 3D blood-brain barrier model prepared with CCF-STTG1 astrocyte cells, pericytes and human umbilical vein endothelial cells (HUVECs), as described in Molinari et al., "The Role of BDNF on Aging-Modulation Markers", Brain Sci. 2020 May; 10(5): 285.

The following analyses were conducted at the level of this compartment:

- Cell viability via an MTT assay
- ROS production rate via cytochrome C
- Integrity of the blood-brain barrier assessed using specific ELISA kits for detecting the main tight junctions (TJs), Claudin-5 and MARVELD
- Analysis of the absorption rate (15'-30'-1h-3h-12h-24h) using a fluorescent probe

In the second phase of the experiment, the main molecular pathways correlated with the triggering of pain transmission and the manifestation of the analgesic effect at the level of the central nervous system were evaluated in a brain organoid placed under conditions of damage after a 30 min pretreatment with 200 $\mu$M of $H_2O_2$.

In order to assess the effect of the samples indicated in Table 1 at the level of the peripheral nervous system, experiments were carried out on a 3D peripheral nerve model indicated as "engineered neural tissue" (EngNT) placed in a condition of neuropathic pain by means of 14 days of pretreatment with GGF 200 ng/mL.

The pretreated cells were stimulated with the supernatants collected in the previous phase after passage along the blood-brain barrier *in vitro.* The following analyses were carried out:

- Analysis of FAAH activity
- Analysis of PPAR$\alpha$ activity
- Analysis of pPKA expression
- Analysis of pTRPV1 expression
- Analysis of CNR2 activity. The appended figure shows the levels of CNR2 present at the cellular level after 24h of treatment.

**Example 1 - experiments on an in vitro 3D blood-brain barrier model (Phase 1)**

Cell viability and ROS production rate

After 24h of treatment with the samples under examination, it was possible to observe that:

- All the substances improved cell viability compared to the control (p<0.05). Better data were found after the combination of *Equisetum A.L.* with Gastrodia, Agaricus and Chaga as compared to *Equisetum A.L.* alone (p<0.05).
- The data relating to cell viability were confirmed by the analysis of ROS production. All the substances reduced the % of ROS compared to the control (p<0.05). Better data were found after the combination of *Equisetum A.L.* with all the substances under examination.
- The combinations of *Equisetum A.L* 10% + Gastrodia and *Equisetum A.L* 10% + Agaricus showed viability and ROS production data almost coinciding with those which emerged after stimulation with ibuprofen alone.
- The combination of *Equisetum A.L* + Chaga demonstrated a better potential for maintaining and increasing cell viability and a better antioxidant potential compared to the other combinations and ibuprofen alone, with statistically significant data versus ibuprofen (p<0.05).

Figure 1 shows the cell viability and ROS production rate obtained after 24 hours of treatment of the in vitro 3D blood-brain barrier model prepared with CCF-STTG1 astrocyte cells, pericytes and human umbilical vein endothelial cells (HUVECs) with the samples tested in example 1 and indicated in the figure.

The data are expressed as the mean $\pm$ SD (%) of 5 independent experiments carried out in triplicate, normalised to the control. * p<0.05 vs control; $\alpha$ p<0.05 vs *Equisetum A.L.* 10%; $\beta$ p<0.05 vs ibuprofen.

State of integrity of the blood-brain barrier

After a 24h treatment with the samples under examination it was possible to observe that:

- All the substances improved the state of activity of the main tight junctions analysed compared to the control (p<0.05). Better data were found after the combination of *Equisetum A.L.* 10% with Gastrodia, Agaricus and Chaga compared to *Equisetum A.L.* 10% (p<0.05).
- The combinations of *Equisetum A.L.* 10% + Gastrodia and *Equisetum A.L.* 10% + Agaricus favoured an increase in the activity of both tight junctions, in a manner nearly coinciding with and slightly greater than that emerging after stimulation with ibuprofen alone.
- The combination of *Equisetum A.L.* 10% + Chaga demonstrated a better potential for maintaining and enhancing blood-brain barrier function and integrity, as reproduced *in vitro,* compared to the other combinations and to ibuprofen alone, with statistically significant data versus ibuprofen (p<0.05).

Figure 2 shows the results relating to the analysis of the state of integrity of the blood-brain barrier, in particular with an assessment of the activity of the tight junction proteins marveld and claudin-5; evaluation after 24 hours of treatment with the samples tested in example 1 and indicated in the figure.
The data are expressed as the mean $\pm$ SD (%) of 5 independent experiments carried out in triplicate, normalised to the control. * p<0.05 vs control; $\alpha$ p<0.05 vs *Equisetum A.L.* 10%; $\beta$ p<0.05 vs ibuprofen.

Analysis of the rate of absorption through the blood-brain barrier

After 24h of treatment with the samples under examination it was possible to observe that:

- All the combinations and ibuprofen demonstrated the same time trend of absorption, with a peak in proximity to 3h of stimulation.
- All the combinations improved the absorption kinetics and the state of permeation along the blood-brain barrier of *Equisetum A.L.* 10% alone (p<0.05). Better data were found after the combination of *Equisetum A.L.* 10% with Chaga, with statistically significant data versus all the other agents under examination (p<0.05).

Figure 3 shows the rate of absorption of the analysed samples through the blood-brain barrier. The data are expressed as the mean $\pm$ SD (%) of 5 independent experiments carried out in triplicate, normalised to the control.
All the combinations and ibuprofen p<0.05 vs control; * p<0.05 vs all the other agents.
In particular, the percentage values of absorption through the blood-brain barrier obtained for the samples tested were calculated according to a method validated in the literature, applying the following formula:

$$J = J_{max} \, [C]/(K_t + [C])$$

C: initial fluorescein concentration
$J_{max}$: maximum permeation rate
$K_t$: Michaelis-Menten constant.

**Example 2 - experiments on a brain organoid and on a 3D peripheral nerve model, EngNT (Phase 2)**

2.1 Analysis of the main molecular pathways correlated to the triggering of pain transmission (CNS)

In order to assess the main molecular pathways correlated to the triggering of pain transmission and the manifestation of the analgesic effect at the level of the central nervous system, the activity of FAAH, PPAR$\alpha$, pPKA and pTRPV1 was assessed in a brain organoid placed under conditions of damage after a 30 min pretreatment with 200 $\mu$M of $H_2O_2$.

Analysis of FAAH activity

It was observed that:

- All the substances were capable of reducing the state of activity of FAAH, which is directly involved in the state of transmission of neuropathic pain at the central level. The substances were capable of reducing its activity versus $H_2O_2$

(p<0.05).

- The combination of *Equisetum A.L.* 10% + Gastrodia favoured an inhibitory effect similar to and slightly smaller than the one emerging after stimulation with ibuprofen alone. Furthermore, *Equisetum A.L.* 10% + Agaricus demonstrated similar effects, slightly smaller than those of *Equisetum A.L.* 10% + Gastrodia and ibuprofen.
- Chaga in combination with *Equisetum A.L.* 10% produced the best data in terms of reducing FAAH activity.

Figure 4 shows the FAAH activity detected after treatment of an organoid, previously treated with 200 $\mu$M of $H_2O_2$ for 30 min, with the samples indicated in the figure. FAAH activity was evaluated by means of a specific kit aimed at assessing the activity thereof; therefore, the graphs in the figure show the activity.

The data are expressed as the mean $\pm$ SD (%) of 5 independent experiments carried out in triplicate, normalised to the control. * p<0.05 vs control; $\alpha$ p<0.05 vs $H_2O_2$ 200 $\mu$M.

Analysis of PPAR$\alpha$, pTRPV1 and pPKA activity

It was observed that:

- All the combinations under examination were capable of favouring an analgesic effect by modulating the markers involved at the level of the CNS.
- PPAR$\alpha$ is widely activated in the analgesic responses at the level of the CNS, and all the combinations favoured an increase thereof versus $H_2O_2$ (p<0.05). Chaga, in combination with *Equisetum A.L.* 10%, showed the best data in terms of amplifying PPAR$\alpha$ activity, in a significant manner compared to ibuprofen (p<0.05).
- pPKA and pTRPV1 represent two directly correlated markers, since, under conditions of pain, more activated pPKA favours the phosphorylation of TRPV1 to pTRPV1, causing the triggering of the pain transmission pathway at the level of the CNS. Consistently with the previous data, they favoured a decrease in the expression of both proteins versus $H_2O_2$ (p<0.05). Chaga, in combination with *Equisetum A.L.* 10%, showed the best data in terms of reducing pPKA and pTRPV1 activity.

- In all three graphs, it is possible to observe that the combinations of *Equisetum A.L* 10% + Gastrodia and *Equisetum A.L* 10% + Agaricus favoured similar effects, almost coinciding with the ones emerging after stimulation with ibuprofen alone (PPAR$\alpha$) and also with the combination *Equisetum A.L.* 10% + Chaga (pTRPV1 and pPKA).

Figure 5 shows the levels of PPAR$\alpha$, pTRPV1 and pPKA detected after treatment of an organoid, previously treated with 200 $\mu$M of $H_2O_2$ for 30 min, with the samples indicated in the figure. As regards PPAR$\alpha$, the graphs show the levels of this protein following treatment with the samples for 24h; whereas as regards pTRPV1 and pPKA, the graphs show the expression of these proteins after 24h of treatment.

The data are expressed as the mean $\pm$ SD (%) of 5 independent experiments carried out in triplicate, normalised to the control. * p<0.05 vs control; $\alpha$ p<0.05 vs $H_2O_2$ 200 $\mu$M; $\beta$ p<0.05 vs *Equisetum A.L.* 10%; $\gamma$ p<0.05 vs ibuprofen.

2.2 Analysis of the main molecular pathways correlated to the triggering of pain transmission (PNS)

In order to assess the main molecular pathways correlated to the triggering of pain transmission and the manifestation of the analgesic effect at the level of the peripheral nervous system, the activity of FAAH, PPAR$\alpha$, pPKA, pTRPV1, and CNR2 was assessed in a 3D peripheral nerve model placed under a condition of neuropathic pain by means of pretreatment with GGF 200 ng/mL for 14 days.

Analysis of FAAH activity

It was observed that:

- All the substances were capable of reducing the state of activity of FAAH, which is directly involved in the state of transmission of neuropathic pain at the central level. The substances were capable of reducing its activity versus GGF 200 ng/mL (p<0.05). Chaga, in combination with *Equisetum A.L.* 10%, showed the best data in terms of reducing FAAH activity.
- The combination of *Equisetum A.L* 10% + Gastrodia and *Equisetum A.L* 10% + Agaricus favoured an inhibitory effect similar to and slightly smaller than the one emerging after stimulation with ibuprofen alone.

Figure 6 shows the FAAH levels detected after treatment of a 3D peripheral nerve model, previously treated with GGF 200 ng/mL for 14 days, with the samples indicated in the figure. The data are expressed as the mean $\pm$ SD (%) of 5

independent experiments carried out in triplicate, normalised to the control. * p<0.05 vs control; $\alpha$ p<0.05 vs GGF 200 ng/Ml.

Analysis of PPAR$\alpha$, pTRPV1, CNR2 and pPKA activity

It was observed that:

- All the combinations under examination were capable of favouring an analgesic effect by modulating the markers involved at the level of the PNS.
- PPAR$\alpha$ is widely activated in the analgesic responses, and all the combinations favoured an increase thereof versus GGF 200 ng/mL and compared to *Equisetum A.L.* 10% alone (p<0.05). Chaga, in combination with *Equisetum A.L.* 10%, showed the best data in terms of amplifying the activity of PPAR$\alpha$, in a significant manner compared to ibuprofen (p<0.05).
- pPKA and pTRPV1 represent two directly correlated markers, since under conditions of pain, more activated pPKA favours the phosphorylation of TRPV1, causing the triggering of the pain transmission pathway at the nerve level. Consistently with the previous data, the combinations favoured a decrease in the expression of both proteins versus GGF 200 ng/mL and compared to *Equisetum A.L.* 10% alone (p<0.05). Chaga, in combination with *Equisetum A.L.* 10%, showed the best data in terms of reducing pPKA and pTRPV1 activity.
- CNR2, directly involved with analgesic responses, was widely activated by all the combinations, but in particular by the combination between Chaga and *Equisetum A.L.* 10% compared to ibuprofen (p<0.05).
- In all four graphs, it is possible to observe that the combinations of *Equisetum A.L* 10% + Gastrodia and *Equisetum A.L* 10% + Agaricus favoured effects similar to and in some cases almost coinciding with those emerging after stimulation with ibuprofen alone (PPAR$\alpha$ and CNR-2) and also with the combination of *Equisetum A.L.* 10% + Chaga (pTRPV1 and pPKA).

Figure 7 shows the levels of PPAR$\alpha$, pTRPV1, CNR2 and pPKA detected after treatment of a 3D peripheral nerve model, previously treated with GGF 200 ng/mL for 14 days, with the samples indicated in the figure.
The data are expressed as the mean $\pm$ SD (%) of 5 independent experiments carried out in triplicate, normalised to the control. * p<0.05 vs control; $\alpha$ p<0.05 vs $H_2O_2$ 200 $\mu$M; $\beta$ p<0.05 vs *Equisetum A.L.* 10%; $\gamma$ p<0.05 vs ibuprofen.

## Conclusions

Based on the study conducted, it is possible to affirm that, among the other things found:

- All the combinations made it possible to increase the state of maintenance and enhance blood-brain barrier function and the absorption kinetics of *Equisetum A.L.* alone (p<0.05).
- Among the combinations under examination, *Equisetum A.L.* 10% 50$\mu$g/mL + Chaga 250 $\mu$g/mL demonstrated biological effects at the level of the blood-brain barrier with statistically significant data versus ibuprofen (p<0.05).
- All the combinations examined represent valid alternatives in the treatment of pain thanks to strong analgesic effect demonstrated by the combination with *Equisetum A.L.* as manifested in all the formulations, at the level of both the CNS and PNS.
- This suggests a crucial analgesic role of *Equisetum A.L.*, which also amplifies the effect of the combined substances in terms of reducing FAAH activity and the expression of pPKA and pTRPV1 at the level of the CNS and PNS, making them capable of acting like ibuprofen.
- Among the combinations, *Equisetum A.L.* 10% + Chaga 250 $\mu$g/mL demonstrated analgesic effects at the nerve level with statistically significant data versus ibuprofen. Furthermore, in all the situations examined, the *Equisetum A.L* 10% + Gastrodia and *Equisetum A.L* 10% + *Agaricus* combinations favoured effects similar to and nearly coinciding with those examined after stimulation with ibuprofen and in some cases also with the combination *Equisetum A.L.* 10% + Chaga 250 $\mu$g/mL.

## Claims

1. A composition comprising an extract of a plant belonging to the genus *Equisetum* and at least one further ingredient selected from the group consisting of:

    i) an extract of a plant belonging to the genus *Gastrodia,* preferably to the species G. *elata,*
    ii) an extract of a fungus belonging to the species *Agaricus blazei Murrill* or *Inonotus obliquus* (Chaga) and mixtures thereof.

2. The composition according to claim 1, wherein said composition comprises or consists of an extract of the plant belonging to the genus *Equisetum* and an extract of the fungus belonging to the species *Inonotus obliquus* (Chaga).

3. The composition according to claim 1, wherein said composition comprises or consists of an extract of the plant belonging to the genus *Equisetum* and an extract of the fungus belonging to the species *Agaricus blazei Murrill.*

4. The composition according to claim 1, wherein said composition comprises or consists of an extract of the plant belonging to the genus *Equisetum* and an extract of a plant belonging to the genus *Gastrodia.*

5. The composition according to any one of claims 1-4, wherein said extract of the plant belonging to the genus *Equisetum* belongs to the species *Equisetum L. arvense.*

6. The composition according to any one of claims 1-5, wherein the composition comprises the extract of a plant belonging to the genus *Equisetum* and the extract of the fungus belonging to the species *Inonotus obliquus* (Chaga) in a weight ratio by weight ranging between 1:10 and 1:1, preferably between 1:8 and 1:3.

7. The composition according to any one of claims 1-6, wherein said composition comprises the extract of a plant belonging to the genus *Equisetum* and the extract of the fungus belonging to the species *Agaricus blazei Murrill* in a weight ratio by weight ranging between 1:10 and 1:1, preferably between 1:8 and 1:3.

8. The composition according to any one of claims 1-7, wherein said composition comprises the *Equisetum* extract and the Gastrodia extract in a weight ratio by weight ranging between 1:10 and 2:1, preferably between 1:5 and 1:1.

9. The composition according to any one of claims 1-8, formulated as granules, a tablet, capsule, sachet, stick, orodispersible stick, gel, pill, oral ampoule, soluble powder, or syrup.

10. The composition according to any one of claims 1-9, for use as a medicament.

11. The composition according to any one of claims 1-9, for use in the treatment or prevention of pain.

12. The composition for use according to claim 11, wherein the pain is a chronic pain and/or an acute pain.

13. The composition for use according to claim 11 or 12, wherein the pain is a nociceptive pain, a neuropathic pain and/or a dysfunctional pain, and wherein the neuropathic pain is a central or peripheral neuropathic pain.

14. The composition for use according to any one of claims 11-13, in association or in combination with at least one active ingredient or a composition for treating pain and/or inflammation in an individual.

15. A dietary supplement comprising or consisting of the composition according to any one of claims 1-9.

Fig. 1

A

B

Fig. 2

Fig. 3

Fig. 4

Fig. 5

Fig. 6

Fig. 7

Fig. 7

Europäisches
Patentamt

European
Patent Office

Office européen
des brevets

## EUROPEAN SEARCH REPORT

**Application Number**

EP 25 19 8615

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2019 014714 A (EARTH CHEMICAL CO) 31 January 2019 (2019-01-31) * claims 1-2; figure 1; examples 1-4 * ----- | 1-15 | INV. A61K36/8988 A61K36/11 A61K36/07 A61K31/714 |
| X | JP 2011 207814 A (CCI CORP; GIFU PREFECTURE KENKYU KAIHATSU ZAIDAN) 20 October 2011 (2011-10-20) * paragraphs [0017], [0020]; example 1 * ----- | 1-15 | A61K36/074 A23L33/105 A61P25/00 A61P25/28 A61P29/00 A61K36/45 |
| X | Anonymous: "Natural Botanical Extracts", Carrubba, 26 February 2007 (2007-02-26), pages 1-247, XP055151596, Retrieved from the Internet: URL:http://www.carrubba.com/pdf/Carrubba_Botanical_Guide_r.pdf [retrieved on 2014-11-07] * page 165 * ----- | 1-15 | |
| X | WHO: "Medicinal Plants in the Republic of Korea", Western pacific Series No. 21, 1 January 1998 (1998-01-01), pages 1-329, XP055692830, Manila, Philippines Retrieved from the Internet: URL:https://apps.who.int/iris/bitstream/handle/10665/207585/9290611200_eng.pdf?sequnce=1&isAllowed=y [retrieved on 2020-05-07] * page 129 * ----- | 1-15 | **TECHNICAL FIELDS SEARCHED (IPC)** A61K A23L A61P |

-/--

The present search report has been drawn up for all claims

| Place of search | Date of completion of the search | Examiner |
|---|---|---|
| Munich | 14 January 2026 | Rodrigo-Simón, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................................

& : member of the same patent family, corresponding document

EPO FORM 1503 03.82 (P04C01)

page 1 of 3

Europäisches
Patentamt

European
Patent Office

Office européen
des brevets

## EUROPEAN SEARCH REPORT

**Application Number**

EP 25 19 8615

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HALDER SWATI ET AL: "Herbal drugs and natural bioactive products as potential therapeutics: A review on pro-cognitives and brain boosters perspectives", SAUDI PHARMACEUTICAL JOURNAL, ELSEVIER, AMSTERDAM, NL, vol. 29, no. 8, 15 July 2021 (2021-07-15), pages 879-907, XP086725669, ISSN: 1319-0164, DOI: 10.1016/J.JSPS.2021.07.003 [retrieved on 2021-07-15] * page 891, column 1, paragraph 6 * | 1-15 | |
| X | ZHOU HAI-BO ET AL: "Mechanisms for the biological activity of Gastrodia elata Blume and its constituents: A comprehensive review on sedative-hypnotic, and antidepressant properties", PHYTOMEDICINE, ELSEVIER, AMSTERDAM, NL, vol. 123, 28 November 2023 (2023-11-28), XP087451641, ISSN: 0944-7113, DOI: 10.1016/J.PHYMED.2023.155251 [retrieved on 2023-11-28] * abstract; table 2 * | 1-15 | TECHNICAL FIELDS SEARCHED (IPC) |
| X | KOMSIT WISITRASSAMEEWONG ET AL: "Agaricus subrufescens: A review", SAUDI JOURNAL OF BIOLOGICAL SCIENCES, vol. 19, no. 2, 1 April 2012 (2012-04-01), pages 131-146, XP055644754, AMSTERDAM, NL ISSN: 1319-562X, DOI: 10.1016/j.sjbs.2012.01.003 * table 2 * | 1-15 | |

-/--

The present search report has been drawn up for all claims

| Place of search | Date of completion of the search | Examiner |
|---|---|---|
| Munich | 14 January 2026 | Rodrigo-Simón, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

Europäisches
Patentamt

European
Patent Office

Office européen
des brevets

**EUROPEAN SEARCH REPORT**

Application Number

EP 25 19 8615

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 2018 0003002 A (UNIV HANNAM INST IND ACAD COOP [KR]; IAC IN NAT UNIV CHUNGNAM [KR]) 9 January 2018 (2018-01-09) * claims 1-8 * | 1-15 | |
| X | CN 113 975 305 A (ZHANG ZHIZHONG) 28 January 2022 (2022-01-28) * page 1, paragraph 1; claims 1-3 * | 1-15 | |
| X | HETLAND GEIR ET AL: "Antitumor, Anti-inflammatory and Antiallergic Effects of Agaricus blazei Mushroom Extract and the Related Medicinal Basidiomycetes Mushrooms, Hericium erinaceus and Grifola frondosa: A Review of Preclinical and Clinical Studies", NUTRIENTS, vol. 12, no. 5, 1 May 2020 (2020-05-01), page 1339, XP093353168, CH ISSN: 2072-6643, DOI: 10.3390/nu12051339 * abstract; table 5 * | 1-15 | TECHNICAL FIELDS SEARCHED (IPC) |

The present search report has been drawn up for all claims

| Place of search | Date of completion of the search | Examiner |
|---|---|---|
| Munich | 14 January 2026 | Rodrigo-Simón, Ana |

EPO FORM 1503 03.82 (P04C01)

## ANNEX TO THE EUROPEAN SEARCH REPORT
## ON EUROPEAN PATENT APPLICATION NO.

EP 25 19 8615

This annex lists the patent family members relating to the patent documents cited in the above-mentioned European search report.
The members are as contained in the European Patent Office EDP file on
The European Patent Office is in no way liable for these particulars which are merely given for the purpose of information.

14-01-2026

| Patent document cited in search report | | Publication date | Patent family member(s) | | Publication date |
|---|---|---|---|---|---|
| JP 2019014714 | A | 31-01-2019 | JP 7553620 | B2 | 18-09-2024 |
| | | | JP 7596059 | B2 | 09-12-2024 |
| | | | JP 2019014714 | A | 31-01-2019 |
| | | | JP 2023052833 | A | 12-04-2023 |
| | | | JP 2024173940 | A | 13-12-2024 |
| JP 2011207814 | A | 20-10-2011 | NONE | | |
| KR 20180003002 | A | 09-01-2018 | NONE | | |
| CN 113975305 | A | 28-01-2022 | NONE | | |

EPO FORM P0459

For more details about this annex : see Official Journal of the European Patent Office, No. 12/82

# EP 4 702 984 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Remington: The Science and Practice of Pharmacy. Lippincott, Williams & Wilkins

- **MOLINARI et al.** The Role of BDNF on Aging-Modulation Markers. *Brain Sci.*, May 2020, vol. 10 (5), 285